(19) Europäisches Patentamt
European Patent Office
Office européen des brevets

(11) Veröffentlichungsnummer: **0 253 182**
**A1**

# (12) EUROPÄISCHE PATENTANMELDUNG

(21) Anmeldenummer: 87109273.0

(22) Anmeldetag: 27.06.87

(51) Int. Cl.4: **C07D 285/16**, A61K 31/54

(30) Priorität: **12.07.86 DE 3623532**

(43) Veröffentlichungstag der Anmeldung:
**20.01.88 Patentblatt 88/03**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Müller, Nikolaus, Dr.**
**Knipprather Strasse 98**
**D-4019 Monheim(DE)**
Erfinder: **Andrews, Peter, Dr.**
**Gellertweg 2**
**D-5600 Wuppertal 1(DE)**

## (54) Verwendung von Thiadiazinonen zur Bekämpfung von Endoparasiten.

(57) Die vorliegende Erfindung betrifft die Verwendung von Thiadiazinonen der Formel I

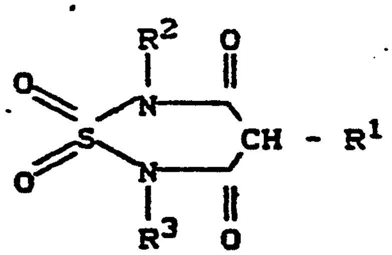

I

in welcher
$R^1$ für den Rest der Formel steht,

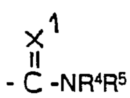

wobei
$X^1$ für O oder S steht,
$R^4$ für Wasserstoff oder Alkyl steht,
$R^5$ für gegebenenfalls substituiertes Phenyl steht,
$R^2$ für Wasserstoff, Alkyl, Cycloalkyl, Aryl oder Aralkyl steht,
$R^3$ für Wasserstoff, Alkyl, Cycloalkyl, Aryl oder Aralkyl steht,
zur Bekämpfung von Endoparasiten in der Veterinärmedizin sowie neue Thiadiazinone der Formel I und ihre Herstellung.

EP 0 253 182 A1

## Verwendung von Thiadiazinonen zur Bekämpfung von Endoparasiten

Die vorliegende Erfindung betrifft die Verwendung von Thiadiazinonen zur Bekämpfung von Endoparasiten in der Veterinärmedizin.

Die Verwendung von Thiadiazinonen als Fungizide bei Pflanzen ist bereits bekannt geworden (DE-OS 3 230 923). Es ist jedoch nichts über ihre Eignung zur Bekämpfung von Endoparasiten, insbesondere von Helminthen, bekannt geworden.

1. Es wurde gefunden, daß Thiadiazinone der Formel I

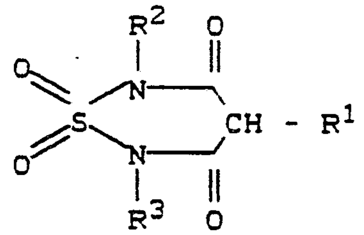

I

in welcher

$R^1$ für den Rest der Formel steht,

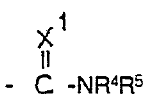

wobei

$X^1$ für O oder S steht,

$R^4$ für Wasserstoff oder Alkyl steht,

$R^5$ für gegebenenfalls substituiertes Phenyl steht,

$R^2$ für Wasserstoff, Alkyl, Cycloalkyl, Aryl oder Aralkyl steht,

$R^3$ für Wasserstoff, Alkyl, Cycloalkyl, Aryl oder Aralkyl steht,

sich hervorragend zur Bekämpfung von Endoparasiten in der Verterinärmedizin eignen.

Die Verbindungen der Formel 1 können in Form ihrer verschiedenen Tautomeren (Keto/Enol) sowie als Gemische dieser Tautomeren, sowie in Form ihrer Salze mit Basen vorliegen.

Die Verbindungen der Formel I sind bekannt oder lassen sich analog zu einem der weiter unten angegebenen bekannten Verfahren herstellen.

2. Thiadiazinone der Formel II sind neu

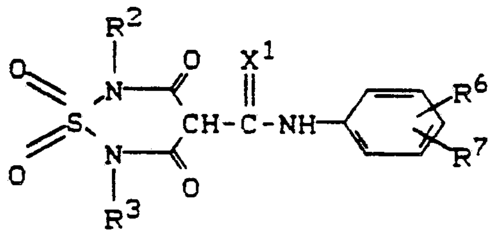

II

in welcher

$R^2$, $R^3$ und $X^1$ die oben angegebene Bedeutung besitzen,

$R^6$ für $SCF_3$, gegebenenfalls substituiertes Phenoxy oder Pyridyloxy steht,

$R^7$ für Wasserstoff, Halogen, insbesondere Chlor, steht.

3. Thiadiazinone der Formel II

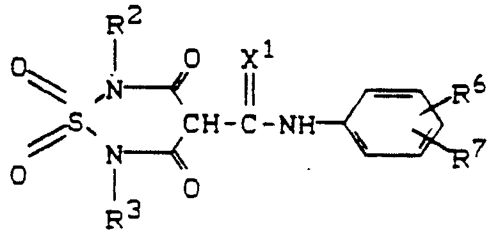

II

in welcher
$R^2$, $R^3$ und $X^1$ die oben angegebene Bedeutung besitzen,
$R^6$ für $SCF_3$, gegebenenfalls substituiertes Phenoxy oder Pyridyloxy steht,
$R^7$ für Wasserstoff, Halogen, insbesondere Chlor, steht.
werden hergestellt, indem man
a) Thiadiazinone der Formel III

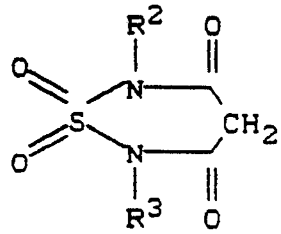

III

in welcher
$R^2$, $R^3$ die oben angegebene Bedeutung haben, mit Isocyanaten der Formel IV

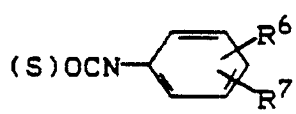

IV

in welcher
$R^6$ und $R^7$ die oben angegebene Bedeutung haben gegebenenfalls in Gegenwart von Katalysatoren umsetzt, oder indem man
b) Thiadiazinone der Formel V

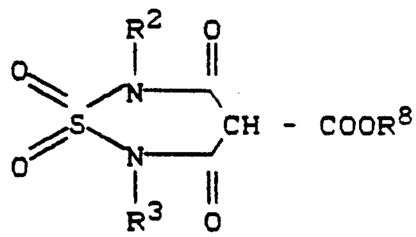

V

in welcher
$R^2$, $R^3$ die oben angegebene Bedeutung haben,
$R^8$ für $C_{1-4}$-Alkyl steht,
mit Aminen der Formel V
$HNR^4R^5$ VI
in welcher
$R^4$ und $R^5$ die oben angegebene Bedeutung haben umsetzt.

Bevorzugt sind Verbindungen der Formel I in welcher
$R^2$ für Wasserstoff, $C_1$-$C_4$-Alkyl, $C_{3-8}$-Cycloalkyl, Phenyl, Benzyl, die gegebenenfalls substituiert sein können, steht,
$R^3$ für Wasserstoff, $C_1$-$C_4$-Alkyl, $C_{3-8}$-Cycloalkyl, Phenyl, Benzyl, die gegebenenfalls substituiert sein können,

steht,

$R^1$ für den Rest -CO-$NR^4R^5$ steht,

$R^4$ für Wasserstoff oder $C_{1-4}$-Alkyl steht,

$R^5$ für Phenyl steht, das gegebenenfalls einen oder mehrere, gleiche oder verschiedene der folgenden Substituenten trägt:

Alkyl mit vorzugsweise 1 bis 4, insbesondere 1 oder 2 Kohlenstoffatomen, wie Methyl, Ethyl, n.-und i.-Propyl und n.-, i.-und t.-Butyl; Alkoxy mit vorzugsweise 1 bis 4, insbesondere 1 oder 2 Kohlenstoffatomen, wie Methoxy, Ethoxy, n.-und i.-Propyloxy und n.-, i.-und t.-Butyloxy; Alkylthio mit vorzugsweise 1 bis 4, insbesondere 1 oder 2 Kohlen stoffatomen, wie Methylthio, Ethylthio, n.-und i.-Propylthio und n.-, i.-und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise, Fluor, Chlor, Brom, insbesondere Fluor stehen, wie Trifluormethylthio; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl-und Dialkyl amino mit vorzugsweise 1 bis 4, insbesondere 1 oder 2 Kohlenstoffatomen je Alkylgruppe, wie Methylamino, Methyl-ethyl-amino, n.- und i.-Propylamino und Methyl-n.-Butylamino; Formyl; Carboxyl; Carbalkoxy mit vorzugsweise 2 bis 4, insbesondere 2 oder 3 Kohlenstoffatomen, wie Carbomethoxy und Carboethoxy; Sulfo (-$SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Heteroaryloxy wie z. B. Pyridyloxy, die ihrerseits wieder substituiert sein können.

Besonders bevorzugt sind Verbindungen der Formel I, in welcher

$R^2$ für $C_1$-$C_4$-Alkyl, Cyclohexyl, Phenyl, das gegebenenfalls durch Halogen substituiert ist, oder Benzyl steht,

$R^3$ für $C_1$-$C_4$-Alkyl, Cyclohexyl, Phenyl, das gegebenenfalls durch Halogen substituiert ist, oder Benzylsteht,

$R^1$ für den Rest

$$-\overset{\overset{\displaystyle O}{\|}}{C}-NHR^5$$

steht, wobei

$R^5$ für Phenyl steht, das gegebenenfalls ein-oder mehrfach, gleich oder verschieden substituiert ist durch $C_1$-$C_4$-Alkyl, insbesondere Methyl, $C_1$-$C_4$-Alkoxy, insbesondere Methoxy, Ethoxy, $C_1$-$C_4$-Halogenalkoxy, insbesondere Trifluormethoxy, Fluorchlorethoxy, $C_1$-$C_4$-Halogenalkylthio, insbesondere Trifluormethylthio, Fluorchlormethylthio, $C_1$-$C_4$-Alkylthio, insbesondere Methylthio, Halogensulfonyl, insbesondere Fluorsulfonyl, Chlorsulfonyl, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Methylendioxy oder Ethylendioxy, die gegebenenfalls durch Fluor oder Chlor substituiert sind, Halogen, insbesondere Fluor oder Chlor, $NO_2$, Phenoxy oder Pyridyloxy, die gegebenenfalls durch einen der oben angegebenen Reste substituiert sind.

Ganz besonders bevorzugt sind Verbindungen der Formel I in welcher

$R^2$ für $C_1$-$C_4$-Alkyl insbesondere Methyl oder Ethyl, Cyclohexyl, Phenyl oder Chlorphenyl steht,

$R^3$ für $C_1$-$C_4$-Alkyl insbesondere Methyl, Ethyl, Propyl, Cyclohexyl oder Chlorphenyl steht,

$R^1$ für den Rest -$CONHR^5$ steht,

$R^5$ für Phenyl steht, gegebenenfalls ein-oder mehrfach, gleich oder verschieden durch Halogen, insbesondere Chlor, $NO_2$, $CF_3$, $OCF_3$, $SO_2F$, $SCF_3$, $SCF_2Cl$, $SOCF_3$, $SO_2CF_3$, $OCH_3$, $OCF_2CF_2H$, Phenoxy oder Pyridyloxy, die durch $CF_3$ oder $OCF_3$ substituiert sind.

Im einzelnen seien folgende Verbindungen der Formel I genannt:

| | | | $R^1 = CX^1NR^4R^5$ | |
|---|---|---|---|---|
| $R^2$ | $R^3$ | $X^1$ | $R^4$ | $R^5$ |
| $C_6H_5$ | $-CH_3$ | O | H | 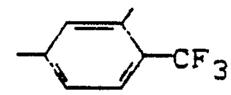 |
| $-C_6H_5$ | $-CH_3$ | S | $C_2H_5$ | 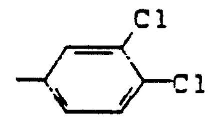 |
| $-C_6H_5$ | $-CH_3$ | O | $CH_3$ | 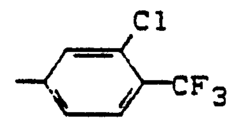 |
| $-CH_3$ | $-CH_3$ | O | H | 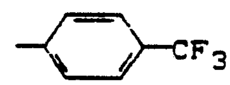 |
| $-CH_3$ | $-CH_3$ | O | $CH_3$ | 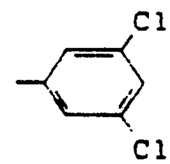 |

Als Basen mit denen die Verbindungen der Formel I Salze bilden können seien genannt: Alkali-und Erdalkali hydroxide, Ammoniak, primäre, sekundäre und tertiäre Amine. Besonders bevorzugt seien folgende Basen genannt:

Triethylamin, Isopropylamin, t-Butylamin, Pyridin, α-Picolin, Trimethylamin, Diisopropylamin, Morpholin, Pyrrolidin, Hexamethylenimin.

Bei den neuen Verbindungen der Formel II sind diejenigen bevorzugt und besonders bevorzugt, in denen die Reste $R^2$ und $R^3$ die beiden Verbindungen der in Formel I angegebenen bevorzugten Bedeutungen besitzen und

$X^1$ für Sauerstoff steht und

$R^6$ für $SCF_3$, Phenoxy oder Pyridyloxy, das durch $CF_3$, $OCF_3$ oder $S\text{-}CF_3$ substituiert ist, und

$R^7$ für Wasserstoff oder Chlor steht.

Im einzelnen seien folgende neue Verbindungen der Formel II genannt:

2,6-Dimethyl-3,5-dioxo-4-[-N-[4-(4-trifluormethyl(phenoxy) -phenylcarbamoyl]]-2H-3,4,5,6-tetrahydro-1,2,6-thiadiazin-1,1-dioxid, 2-Methyl-3,5-dioxo-4-[N-(4-trifluormethylthiolphenylcarbamoyl]-6-ethyl-2H-3,4,5,6-tetrahydro-1,2,6-thiadiazin-1,1-dioxid, 2-Methyl-3,5-dioxo-4-[N-[4-(3-trifluormethylphenoxy)-phenylcarbamoyl]]-2H-3,4,5,6-tetrahydro-1,2,6-thiadiazin-1,1-dioxid, 2-Methyl-3,5-dioxo-4-[N-[4-(4-trifluormethylphenoxy)-phenylcarbamoyl]]-6-(4-chlorphenyl)-2H-3,4,5,6-tetrahydro-1,2,6-thiadiazin-1,1-dioxid, 2-Ethyl-3,5-dioxo-4-[N-[4-trifluormethylthio] phenylcarbamoyl]-6-cyclohexyl-2H-3,4,5,6-tetrahydro-1,2,6-thiadiazin-1,1-dioxid, 2-Methyl-3,5-dioxo-4-[N-[3-chlor-4-trifluormethylthio]phenylcarbamoyl]-6-phenyl-2H-3,4,5,6-tetrahydro-1,2,6-thiadiazin-1,1-dioxid.

Setzt man bei Verfahren 3 a zur Herstellung der neuen Thiadiazinone der Formel II als Verbindung der Formel III 1,3-Dimethyl-2-thia-1,3-diaza-cyclohexan-4,6-dion und als Verbindung der Formel IV p-$SCF_3$-Phenylisocyanat ein, so läßt sich das Verfahren durch folgendes Formelschema wiedergeben:

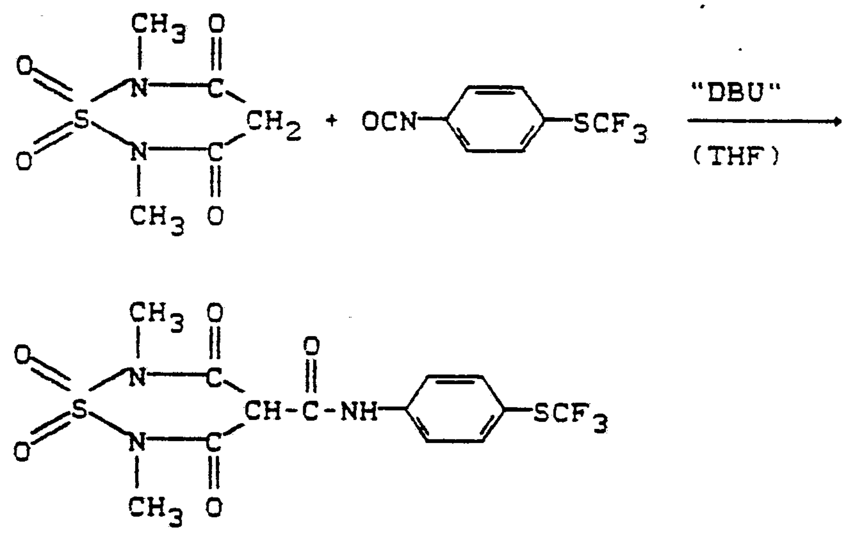


Bevorzugt werden Verbindungen der Formel III eingesetzt, in denen die Substituenten $R^2$, $R^3$ die bei den Verbindungen der Formel I angegebenen bevorzugten und besonders bevorzugten Bedeutungen besitzen. Im einzelnen seien die folgenden Verbindungen der Formel III genannt:

2,6-Dimethyl-3,5-dioxo-2H-3,4,5,6-tetrahydro-1,2,6-thiadiazin-1,1-dioxid, 2-Methyl-6-isopropyl-3,5-dioxo-2H-3,4,5,6-tetrahydro-1,2,6-thiadiazin-1,1-dioxid, 2-Ethyl-6-cyclohexyl-3,5-dioxo-2-H-3,4,5,6-tetrahydro-1,2,6-thiadiazin-1,1-dioxid, 2-Methyl-6-phenyl-3,5-dioxo-2-H-3,4,5,6-tetrahydro-1,2,6-thiadiazin-1,1-dioxid, 2-Isopropyl-6-(4-chlorphenyl)-3,5-dioxo-2-H-3,4,5,6-tetrahydro1,2,6-thiadiazin-1,1-dioxid.

Die Verbindungen der Formel III sind bekannt oder lassen sich analog zu bekannten Verfahren herstellen.

Bevorzugt werden Phenylisocyanate der Formel IV eingesetzt, in denen die Substituenten $R^6$ und $R^7$ die oben angegebenen bevorzugten Bedeutungen haben.

Im einzelnen seien folgende Phenylisocyanate der Formel IV genannt:

Phenylisocyanat, p-Chlorphenylisocyanat, p-Chlorphenylisothiacyanat, 4-Trifluormethylphenylisocyanat, 3-Chlor-4-trifluormethyl-phenylisocyanat, 4-Trifluormethoxyphenylisocyanat, 4-Trifluormethylthiophenylisocyanat, 3,4-Dichlorphenylisocyanat, 4-(4-Trifluormethylphenoxy)-phenylisocyanat 4-(3-Trifluormethylphenoxy)-phenylisocyanat, 4-(5'-Trifluormethyl-2'-pyridyloxy)-phenylisocyanat, 3-Chlor-4-trifluormethoxyphenylisocyanat.

Verbindungen der Formeln III und IV werden in Gegenwart von Verdünnungsmitteln und in Gegenwart von Basen sowie gegebenenfalls in Gegenwart von Katalysatoren umgesetzt.

Als Basen seien genannt: Alkali-, Erdalkalialkoholate und tertiäre Amine. Besonders bevorzugt seien folgende Basen genannt: Triethylamin, Pyridin, Picoline, Trimethylamin, N-Methylmorpholin, N-Ethylpyrrolidin, Diazabicyclo(4,3,0) undecen (DBU), 1,4-Diaza-bicyclo-2,2,2-octan (DABCO), Diazabicyclo(3,2,0)nonen (DBN).

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Katalysatoren kommen die bei Umsetzungen mit Isocyanaten üblichen Katalysatoren infrage. Als solche seien genannt: Metallkatalysatoren des Zn, Sn, Pb wie Dibutylzinndilaurat, Dibutylzinndioxid, Zinnoctoat, Bleioctoat, Zinkoctoat, Zinkchlorid, Zinkacetat.

Die Reaktion wird zwischen 0 und 150°C bevorzugt zwischen 20-50°C durchgeführt. Man arbeitet vorzugsweise unter Normaldruck.

Die Verbindungen der Formeln III und IV werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit verdünnter Säure, Abfiltrieren des Produkts oder Abtrennen der organischen Phase und Abdestillieren des Lösungsmittels.

Setzt man bei Verfahren 3 b zur Herstellung der neuen Thiadiazinone der Formel II als Verbindung der Formel V 1-Phenyl-2-thia-5-carbethoxy-1,3-diazacyclohexan-4,6-dion und als Verbindung der Formel VI 4-Amino-diphenylether ein, so läßt sich das Verfahren durch folgendes Formelschema wiedergeben:

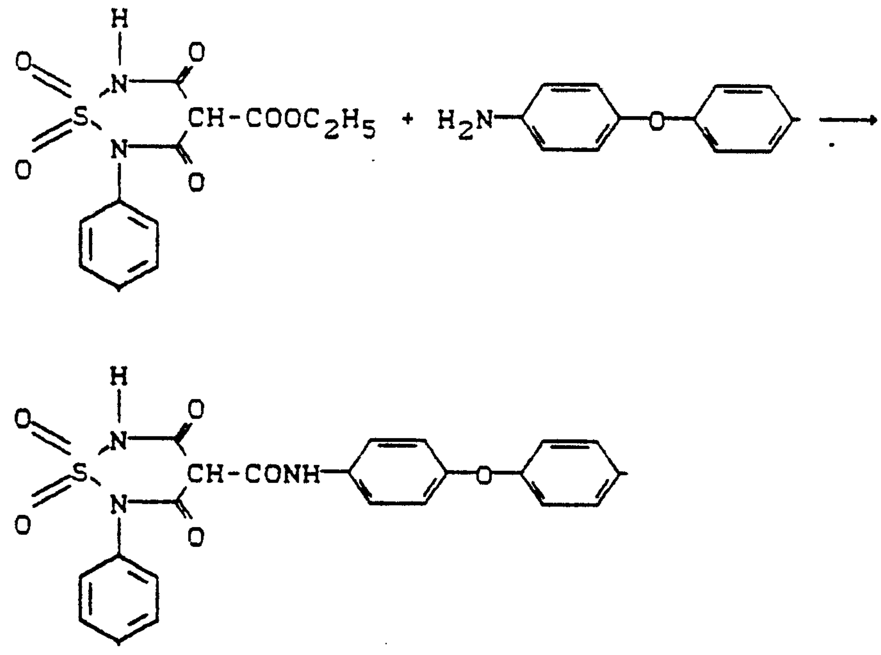

Bevorzugt werden Verbindungen der Formel V eingesetzt, in denen die Substituenten $R^2$ und $R^3$, die bei den Verbindungen der Formel I angegebenen bevorzugten und besonders bevorzugten Bedeutungen besitzen, und $R^8$ für Methyl, Ethyl oder Benzyl steht. Im einzelnen seien folgende Verbindungen der Formel V genannt:

2,6-Dimethyl-4-ethoxycarbonyl-3,5-dioxo-2-H-3,4,5,6-tetrahydro-1,2,6-thiadiazin-1,1-dioxid, 2-Methyl-6-isopropyl-3,5-dioxo-4-methoxycarbonyl-2-H-3,4,5,6-tetrahydro-1,2,6-thiadiazin-1,1-dioxid, 2-Ethyl-6-cyclohexyl-3,5-dioxo-4-ethoxycarbonyl-2-H-3,4,5,6-tetrahydro-1,2,6-thiadiazin-1,1-dioxid, 2-Methyl-6-phenyl-3,5-dioxo-4-methoxycarbonyl-2-H-3,4,5,6-tetrahydro-1,2,6-thiadiazin-1,1-dioxid, 2-Methyl-6-(4-chlorphenyl)-3,5-dioxo-4-ethoxycarbonyl-2-H-3,4,5,6-tetrahydro-1,2,6-thiadiazin-1,1-dioxid, 2,6-Diisopropyl-3,5-dioxo-4-benzyloxycarbonyl-2-H-3,4,5,6-tetrahydro-1,2,6-thiadiazin-1,1-dioxid.

Die Verbindungen der Formel VI sind bekannt oder lassen sich analog nach bekannten Verfahren herstellen.

Die Amine der Formel VI sind bekannt oder lassen sich analog zu bekannten Verfahren herstellen.

Bevorzugt werden Amine der Formel VI eingesetzt, in denen die Substituenten $R^4$ und $R^5$ die bei den Verbindungen der Formel I genannten bevorzugten und besonders bevorzugten Bedeutungen besitzen.

Im einzelnen seien folgende Verbindungen der Formel VI genannt:

3-Chlor-4-trifluormethylanilin, 4-Trifluormethylmercapto-anilin, 3-Chlor-4-trifluormethylmercaptoanilin, 3-Nitro-4-trifluormethylanilin, 2,6-Dichlor-4-trifluormethylmercapto-anilin, 4-Amino-4'-trifluormethyl-diphenylether, 4-Amino-3'-trifluormethyl-diphenylether;

Die Umsetzung der Verbindungen der Formel V und VI erfolgt vorzugsweise in Gegenwart von Verdünnungsmitteln sowie in Gegenwart von Basen.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester

und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid, ferner Alkohole wie Methanol, Ethanol, Propanol, Butanol.

Als Basen seien genannt Alkali-und Erdalkalihydroxide, Alkali-und Erdalkalialkoholate, insbesondere Natriummethylat oder -ethylat.

Die Reaktion wird zwischen 50 und 150°C bevorzugt zwischen 60-110°C durchgeführt. Man arbeitet vorzugsweise unter Normaldruck.

Die Verbindungen der Formeln V und VI werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Abtrennen der organischen Phase und Abdestillieren des Lösungsmittels.

Die erfindungsgemäßen Wirkstoffe sind breit wirksam gegen Endoparasiten. Sie wirken vor allem gegen Cestosen, Trematoden und Nematoden, insbesondere Leberegel und Magen-und Darmnematoden der Wiederkäuer. Darüber hinaus wirken sie auch gegen solche Magen-und Darmnematoden, die gegen die gebräuchlichen Benzimidazol-Anthelmintika resistent und damit nicht mehr ausreichend therapierbar sind.

Die Wirkung wurde im Tierversuch nach oraler, parenteraler und dermaler Applikation bei stark mit Parasiten befallenen Versuchstieren geprüft. Die angewendeten Dosierungen wurden sehr gut von den Versuchstieren vertragen.

Die erfindungsgemäßen Wirkstoffe können als Anthelmintika sowohl in der Human-als auch in der Veterinärmedizin verwendet werden.

Die erfindungsgemäßen Wirkstoffe können zusammen mit anderen üblichen Anthelmintika verabreicht werden.

Die erfingungsgemäßen Wirkstoffe können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können die für eine orale Verabreichung in Betracht kommenden Tabletten und dergleichen mit Süßstoffzusatz und ähnlichen versehen werden. Die therapeutisch wirksame Verbindung soll im vorgenannten Fall in einer Konzentration von etwa 0,5 bis 90 Gew.-% der Gesamtmischung vorhanden sein, d.h. in Mengen, die ausreichend sind, um den obengenannten Dosierungsspielraum zu erreichen.

Die Formulierungen werden in bekannter Weise hergestellt, z.B. durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispeilhaft aufgeführt: Wasser, nicht toxische organische Lösungsmittel, wie Paraffine (z.B. Erdölfraktionen), pflanzliche Öle (z.B. Erdnuß-(Sesamöl), Alkohole (z.B. Ethylalkohol, Glycerin), Glykole (z.B. Propylenglykol, Polyethylenglykol) und Wasser; feste Trägerstoffe, wie z.B. natürliche Gesteinsmehle (z.B. Kaoline, Tonerden, Talkum, Kreide) synthetische Gesteinsmehle (z.B. hochdisperse Kieselsäure, Silikate), Zucker (z.B. Rohr-, Milch-und Traubenzucker); Emulgiermittel, wie nicht ionogene und anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze wie Natriumcitrat, Calciumcarbonat und Dicalciumphosphat, zusammen mit verschiedenen Zuschlagstoffen, wie Stärke, vorzugsweise Karoffelstärke, Gelatine und dergleichen, enthalten. Weiterhin können Gleitmittel, wie Magnesiumstearat, Natriumlaurylsulfat und Talkum, zum Tablettieren mitverwendet werden.

Im Falle wäßriger Suspensionen und/oder Elixieren, die für die orale Anwendung gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen Dragees, Ampullen usw. auch in Form von Dosierungseinheiten sein, wobei jede Dosierungseinheit so angepaßt ist, daß sie eine einzelne Dosis des aktiven Bestandteils liefert.

Die erfindungsgemäßen Wirkstoffe können in den Formulierungen auch in Mischungen mit anderen in der Veterinär-und/oder Humanmedizin zur Behandlung von Infektionen und/oder Erkrankungen benutzten bekannten Wirkstoffen vorliegen, insbesondere L-2,3,5,6-Tetrahydro-6-phenylimidazothiazol, Benzimidazolcarbamaten, Praziquantel, Febantel.

Die Wirkstoffe können in üblicher Weise angewendet werden. Die Applikation erfolgt vorzugsweise oral, eine parenterale, insbesondere subkutane, aber auch dermale Applikationen (pour-on, spot-on) sind möglich.

Im allgemeinen hat es sich als vorteilhaft erwiesen, Mengen von etwa 1 bis etwa 100 mg der Wirkstoffe je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Veterinärmedizin ist der gleiche Dosierungsspielraum vorgesehen. Sinngemäß gelten auch die weiteren obigen Ausführungen.

## Beispiel A

In vitro Nematodentest

Caenorhabditis elegans

Der Wirkstoff wurde in Wasser oder Dimethylsulfoxid (DMSO) gelöst und mit Wasser auf die gewünschte Aufwandkonzentration verdünnt. 0,01 ml dieser Lösung wurde auf eine Replica-Platte gegeben. Dazu wurden 2 ml einer E.coli-Suspension gegeben, zu der man 10-20 weibliche Tiere oder Larven von Caenorhabditis elegans in 0,5 ml sterile M9 Pufferlösung gegeben hat. Die E.coli-Suspension wurde hergestellt indem man 300 ml einer Übernachtkultur eines Uracil-bedürftigen E.coli-Stammes mit 1,8 l steriler M9 Pufferlösung versetzte.

Der Versuchsansatz wurde 7 Tage bei 22°C inkubiert und danach ausgewertet. Es wurde bewertet, inwieweit der Wirkstoff die Vermehrung beeinträchtigt und die Konzentration angegeben, bei der die Vermehrung verhindert wird. Dabei zeigten alle Verbindungen der Herstellungsbeispiele 100 % Wirksamkeit bei einer Aufwandkonzentration von 100 mg/l.

## Beispiel B

In vivo Nematodentest

Heterakis spumosa

Experimentell mit Heterakis spumosa infizierte Mäuse wurden 32 Tage nach der Infektion an vier aufeinanderfolgenden Tagen oral mittels Schlundsonde behandelt. Die Tiere werden 39 Tage nach der Infektion getötet und die Zahl der Parasiten bestimmt. Es wird die Wirkstoffkonzentration angegeben bei der mindestens 95 % der Parasiten abgetötet wurden (effektive Dosis):

| Wirkstoff Beispiel Nr. | effektive Dosis mg/kg |
|---|---|
| 22 | 250 |
| 1 | 100 |
| 3 | 100 |
| 20 | 10 |
| 32 | 25 |

Beispiel C

In vivo Cestodentest

Hymenolepis nana

Experimeltell mit Hymenolepis nana infizierte Mäuse wurden 10 Tage nach der Infektion an vier aufeinanderfolgenden Tagen oral mittels Schlundsonde behandelt. Die Tiere werden 17 Tage nach Infektion getötet und die Zahl der Parasiten bestimmt. Es wird die Wirkstoffkonzentration angegeben, bei der mindestens 95 % der Parasiten abgetötet wurden (effektive Dosis):

| Wirkstoff Beispiel Nr. | effektive Dosis mg/kg |
|---|---|
| 1 | 250 |
| 3 | 100 |
| 18 | 50 |

Beispiel D

In vivo Nematodentest

Strongyloides ratti

Experimentell mit Strongyloides ratti infizierte Ratten wurden 7 Tage nach der Infektion an drei aufeinanderfolgenden Tagen oral mittels Schlundsonde behandelt. Die Tiere werden 13 Tage nach der Infektion getötet und die Zahl der Parasiten bestimmt. Es wird die Wirkstoffkonzentration angegeben. bei der mindestens 95 % der Parasiten abgetötet wurden (effektive Dosis):

| Wirkstoff Beispiel Nr. | effektive Dosis mg/kg |
|---|---|
| 4 | 100 |
| 14 | 100 |
| 28 | 100 |
| 18 | 250 |
| 35 | 25 |
| 33 | 5 |

Beispiel E

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen sind aus der nachfolgenden Tabelle ersichtlich:

Haemonchus contortus / Schaf

| Wirkstoff Beispiel Nr. | effektive Dosis in mg/kg |
|---|---|
| 25 | 5 |
| 26 | 2,5 |
| 28 | 5 |
| 33 | 5 |
| 21 | 10 |
| 20 | 10 |
| 31 | 2,5 |
| 32 | 2,5 |
| 35 | 5 |

Herstellungsbeispiele

a) Allgemeine Vorschrift zur Herstellung der Thiadiazinone gemäß Verfahren 3a)

Je 0,03 Mol des Thiadiazinons der Formel III und des Isocyanats werden in 120 ml trockenem THF gelöst vorgelegt und bei Raumtemperatur innerhalb von 30 min. mit einer Lösung von 0,03 Mol (4,56 g) "DBU" in 30 ml THF versetzt. Die anfänglich hellbraune Lösung verdunkelt sich und es tritt eine leichte Wärmeentwicklung auf, so daß sie Innentemperatur durch Wasserkühlung unter 25°C gehalten werden muß. Anschließend wird bei Raumtemperatur bis zum vollständigen Umsatz nachgerührt (ca. 4-5 h, DC-Probe!). Dann wird der gesamte Ansatz eingedampft, in 100 ml Methylenchlorid aufgenommen und mit 100 ml verdünnter Salzsäure (10 %ig) verrührt. Die organische Phase wird abgetrennt, mit wäßriger Natriumhydrogencarbonatlösung neutral gewaschen und über Natriumsulfat getrocknet. Nach dem Entfernen des Lösungsmittels am Rotationsverdampfer wird der Rückstand aus Ligroin oder Isopropanol umkristallisiert oder chromatographiert.

Analog werden die Wirkstoffe der folgenden Beispiele erhalten:

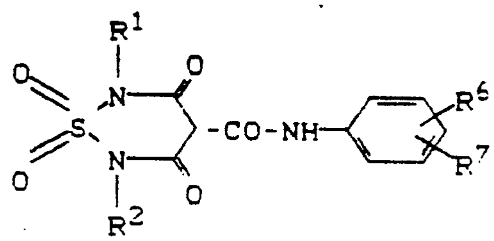

| Bsp. | $R^1$ | $R^2$ | $R^6$ | $R^7$ | Fp |
|---|---|---|---|---|---|
| 1 | $CH_3$ | $iC_3H_7$ | 3-Cl | H | 119° |
| 2 | $CH_3$ | $iC_3H_7$ | $3-CF_3$ | H | 92- 93° |
| 3 | $CH_3$ | $iC_3H_7$ | $4(3'-OC_6H_4CF_3)$ | H | 116-117° |
| 4 | $CH_3$ | $iC_3H_7$ | $4-SCF_3$ | H | 107-109° |
| 5 | $CH_3$ | $iC_3H_7$ | $4(4'-OC_6H_4CF)_3$ | H | 98- 99° |
| 6 | $C_2H_5$ | 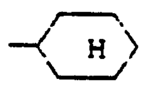 | $4-SCF_3$ | H | 98-100° |
| 7 | $C_2H_5$ | 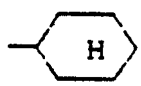 | 3-Cl | H | 128-129° |
| 8 | $C_2H_5$ | 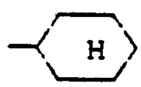 | $4-CF_3$ | H | 128-129° |
| 9 | $C_2H_5$ | 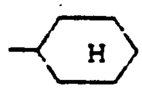 | $4-SCF_3$ | 3-Cl | amorph |
| 10 | $C_2H_5$ | 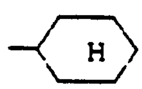 | $4-OCF_3$ | 3-Cl | Oel |
| 11 | $CH_3$ | $C_6H_5$ | 3-Cl | H | 172-173° |
| 12 | $CH_3$ | $C_6H_5$ | $3-CF_3$ | H | 143° |
| 13 | $CH_3$ | $C_6H_5$ | $4-SCF_3$ | H | 211° |
| 14 | $CH_3$ | $C_6H_5$ | $4(3'-OC_6H_4OCF_3)$ | H | 164° |

| Bsp. | $R^1$ | $R^2$ | $R^6$ | $R^7$ | Fp |
|---|---|---|---|---|---|
| 15 | $CH_3$ | $C_6H_5$ | 4-$OCF_3$ | H | 165- 8° |
| 16 | $CH_3$ | $4ClC_6H_4$ | 3-Cl | H | 163° |
| 17 | $CH_3$ | $4ClC_6H_4$ | 3-$CF_3$ | H | 156° |
| 18 | $CH_3$ | $4ClC_6H_4$ | 4-$SCF_3$ | H | 207° |
| 19 | $CH_3$ | $4ClC_6H_4$ | 4-($OC_6H_4$3-$CF_3$) | H | 151° |
| 20 | $CH_3$ | $4ClC_6H_4$ | 4-$CF_3$ | H | 163° |
| 21 | $CH_3$ | $4ClC_6H_4$ | 4-$OCF_3$ | H | 174° |
| 22 | $CH_3$ | Ph | 4-$CF_3$ | H | 225° |
| 23 | $CH_3$ | $CH_3$ | 3-Cl | H | 174-175° |
| 24 | $CH_3$ | $CH_3$ | 3-$CF_3$ | H | 139-140° |
| 25 | $CH_3$ | $CH_3$ | 4-$CF_3$ | H | 145-146° |
| 26 | $CH_3$ | $CH_3$ | 4-$OCF_3$ | H | 112° |
| 27 | $CH_3$ | $CH_3$ | 4-$SCF_3$ | H | 111° |
| 28 | $CH_3$ | $CH_3$ | 4- 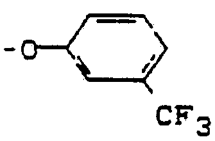 | H | 132° |
| 29 | $CH_3$ | $C_2H_5$ | 3-Cl | H | 159-160° |
| 30 | $CH_3$ | $C_2H_5$ | 3-$CF_3$ | H | 104-105° |
| 31 | $CH_3$ | $C_2H_5$ | 4-$CF_3$ | H | 79- 80° |
| 32 | $CH_3$ | $C_2H_5$ | 4-$OCF_3$ | H | 77° |
| 33 | $CH_3$ | $C_2H_5$ | 4- 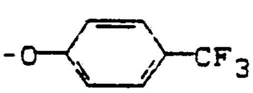 | H | 76- 78° |

| Bsp. | $R^1$ | $R^2$ | $R^6$ | $R^7$ | Fp |
|---|---|---|---|---|---|
| 34 | $CH_3$ | $C_2H_5$ | 4-$SCF_3$ | H | 110-111° |
| 35 | $CH_3$ | $CH_3$ | 4- 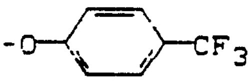 | H | 144-146° |
| 36 | $CH_3$ | $C_2H_5$ | 4- 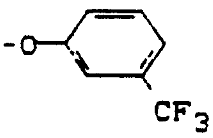 | H | 98-98° |

**Ansprüche**

1. Verwendung von Thiadiazinonen der Formel I

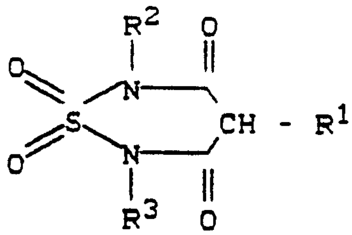

I

in welcher
$R^1$ für den Rest der Formel steht,

$X^1$
‖
$- C -NR^4R^5$

wobei
$X^1$ für O oder S steht,
$R^4$ für Wasserstoff oder Alkyl steht,
$R^2$ für Wasserstoff, Alkyl, Cycloalkyl, Aryl oder Aralkyl steht,
$R^3$ für Wasserstoff, Alkyl, Cycloalkyl, Aryl oder Aralkyl steht,
$R^5$ für gegebenenfalls substituiertes Phenyl steht. zur Bekämpfung von Endoparasiten.

2. Mittel zur Bekämpfung von Endoparasiten, gekennzeichnet durch einen Gehalt an mindestens einem Thiadiazinon der Formel I gemäß Anspruch 1.

3. Verwendung von Thiadiazinonen der Formel I gemäß Anspruch 1 zur Herstellung von Mitteln zur Bekämpfung Endoparasiten.

4. Thiadiazinone der Formel II

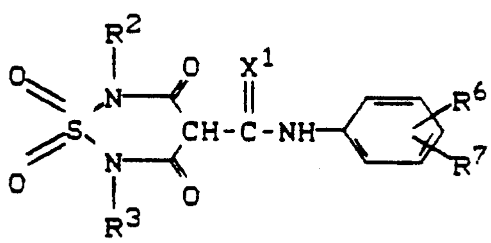

II

in welcher
$X^1$ für O oder S steht,
$R^6$ für $SCF_3$, gegebenenfalls substituiertes Phenoxy steht,
$R^7$ für Wasserstoff, Halogen, insbesondere Chlor, steht,
$R^2$ für Wasserstoff, Alkyl, Cycloalkyl, Aryl oder Aralkyl steht,
$R^3$ für Wasserstoff, Alkyl, Cycloalkyl, Aryl oder Aralkyl steht.

5. Verfahren zur Herstellung von Thiadiazinonen der Formel II

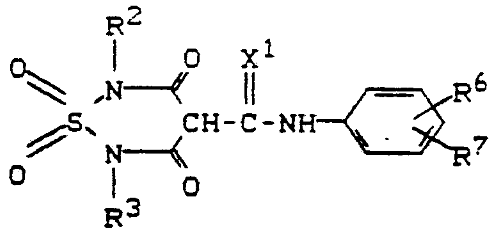

II

in welcher
$X^1$ für O oder S steht,
$R^6$ für $SCF_3$, gegebenenfalls substituiertes Phenoxy steht,
$R^7$ für Wasserstoff, Halogen, insbesondere Chlor, steht,
$R^2$ für Wasserstoff, Alkyl, Cycloalkyl, Aryl oder Aralkyl steht,
$R^3$ für Wasserstoff, Alkyl, Cycloalkyl, Aryl oder Aralkyl steht,
dadurch gekennzeichnet, daß man
a) Thiadiazinone der Formel III

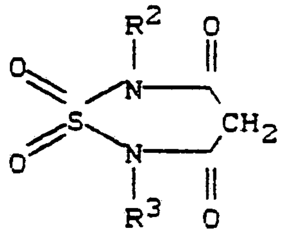

III

in welcher
$R^2$, $R^3$ die oben angegebene Bedeutung haben, mit Isocyanaten der Formel IV

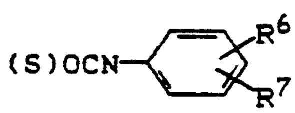

IV

in welcher
$R^6$ und $R^7$ die oben angegebene Bedeutung haben gegebenenfalls in Gegenwart von Katalysatoren umsetzt, oder
b) Thiadiazinone der Formel V

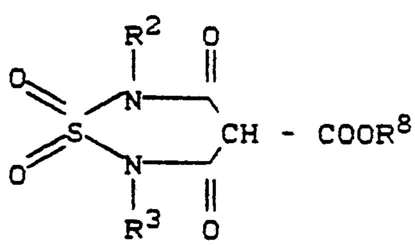

V

in welcher

$R^2$, $R^3$ die oben angegebene Bedeutung haben,
$R^8$ für $C_{1-4}$-Alkyl steht,
mit Aminen der Formel V
$HNR^4R^5$ VI
in welcher
$R^4$ und $R^5$ die oben angegebene Bedeutung haben, umsetzt.

Europäisches Patentamt

# EUROPÄISCHER RECHERCHENBERICHT

Nummer der Anmeldung

EP 87 10 9273

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y | DE-A-2 742 546 (ROUSSEL-UCLAF)<br>* Seiten 29-31 *<br>--- | 1-5 | C 07 D 285/16<br>A 61 K 31/54 |
| Y | CHEMICAL ABSTRACTS, Band 91, Nr. 5, 30. Juli 1979, Seite 641, Zusammenfassung Nr. 39541f, Columbus, Ohio, US; & JP-A-79 05 991 (NIPPON SODA CO. LTD) 17-01-1979<br>* Zusammenfassung *<br>----- | 1-5 | |

| RECHERCHIERTE SACHGEBIETE (Int. Cl.4) |
|---|
| C 07 D 285/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 16-09-1987 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82